# EUROPEAN PATENT SPECIFICATION

(11) **EP 0 391 974 B1**
(45) Date of publication and mention of the grant of the patent: **16.06.1993**
(21) Application number: 89901619.0
(22) Date of filing: 22.12.1988
(51) Int. Cl.: A61K 35/16, A61K 37/02

(54) **Process for the purification of vitamin K-dependent blood clotting factors**
Verfahren zur Reinigung von Vitamin-K-abhängigen Blutgerinnungsfaktoren
Procédé pour la purification des facteurs coagulants du sang dépendants de la vitamine K

(30) Priority: 22.12.1987 GB 8729822
(43) Date of publication of application: 17.10.1990
(73) Proprietor: National Blood Authority, Watford, Herts. WD1 1QH (GB)
(72) Inventor: FELDMAN, Peter, Anthony, Oxford OX4 3PA (GB)
(74) Representative: Holmes, Michael John
(86) International application number: GB8801150
(87) International publication number: WO8905650

(56) References cited:
- US-A- 4 473 553
- AFFINITY CHROMATOGRAPHY, PRINCIPLES AND METHODS, 1986, Pharmacia, Laboratory Separation Division, Uppsala (SE); p. 32#
- THROMBOSIS RESEARCH, vol. 41, no. 1, 1986, Pergamon Press Ltd. (US); R.A.PIXLEY et al., pp. 89-98#
- BIOCHIMICA ET BIOPHYSICA ACTA, vol. 839, 1985, Elsevier Science Publ. BV (Biomedical Division); K.M.WEERASINGHE et al., pp. 57-61#
- MERCK INDEX, 10th ed.; p. 566#
- CLINICA CHIMICA ACTA, vol. 170, 1987; pp. 1-24#

## Description

This invention concerns an improved method for the purification or enrichment of vitamin K-dependent blood clotting factors in fractions containing such factors.

Plasma obtained from blood of human or animal origin contains a number of valuable physiologically active substances, notably the various blood clotting factors, some of which are used in medicine for the treatment of individuals lacking one or more of such substances. The blood clotting factors, i.e. the members of the so-called blood clotting cascade, comprise a group of inactive and active proteolytic enzymes and modified proteins related in the following manner: 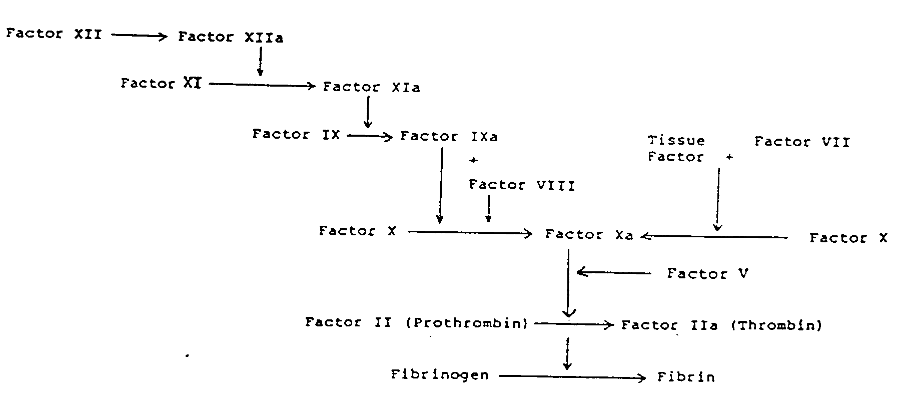
The subscript "a" means that the enzyme is in the activated form.

Further significant plasma proteins, which may be considered to fall within the general category of blood clotting factors, include Protein C and Protein S. Protein C is an inactive zymogen of the proteolytic enzyme Activated Protein C (APC,PCₐ). Unlike the above-mentioned clotting factors, APC has an anticoagulant effect which functions through the proteolysis of Factor V and Factor VIII which are thus inactivated. Activation of Protein C appears to be by a feedback process involving thrombin and an endothelium-bound protein, thrombomodulin, which both function during the coagulation process. The activity of APC is then enhanced by the presence of Protein S, which seems to act as a cofactor.

A number of the above factors, namely II, VII, IX, X, and also Protein C and Protein S, have been shown to be vitamin K-dependent. These factors, both in the active and inactive form (apart from activated Factor II), carry terminal gamma-carboxy glutamic acid residues which are introduced by an enzymic process mediated by vitamin K. Additionally, Protein C, Protein S and Factors X and IX, but not prothrombin (Factor II), have been found to possess a modified aspartic acid residue, namely a β-hydroxyaspartic acid residue.

Thus, the vitamin K-dependent blood clotting factors represent a group of related acidic proteins having closely similar physicochemical properties. The acidic nature of these substances has enabled them previously to be isolated as a group though their structural and chemical similarities have made it difficult to separate the individual proteins using conventional techniques such as anion exchange chromatography.

Apart from Factor VIII, which is not part of the prothrombin complex, the most frequently administered blood clotting factor is Factor IX. Traditionally, Factor IX has been provided as a concentrate of all the factors of the prothrombin complex, II, VII, IX and X, although Factor VII is sometimes provided separately from IX, II and X.

Following infusions of such Factor IX concentrates, there have been a few reported cases of venous thrombosis and disseminated intravascular coagulation (DIC). These undesirable responses might be ascribed to a hypercoagulable state encouraged by high levels of unnecessary clotting factors infused with the Factor IX and/or by unwanted thrombogenic components in the concentrate. Intense and lengthy administration of Factor IX may lead to much higher than normal levels of Factor II, which is infused in the equivalent amounts and has a longer half-disappearance time in plasma. Some hypotheses based on in vitro studies or animal models have also suggested that a combination of factors might be more thrombogenic than Factor IX alone. Indeed, present dosage levels of Factor IX are in part limited by the need to avoid excessive levels of Factor II.

Furthermore, following treatment with factor VIII concentrates, some haemophilia A patients show signs of non-viral immunosuppression. A similar immunosuppression in haemophilia B patients after treatment with Factor IX concentrate suggests that the response in both cases may be due to an unwanted contaminant in the concentrates.

Donor plasma pools from which clotting factor concentrates are prepared contain the causative agent (probably viral) of non-A non-B hepatitis and must be assumed to carry the risk of contamination with viruses (e.g. hepatitis B and HIV). To improve product safety, the concentrates must undergo virucidal treatments, which may be complicated by the presence of contaminant proteins. For example, this virucidal treatment may be easier to devise in a more highly purified concentrate in which interference from unnecessary proteins can be minimised.

In general, it is preferable in principle to administer a blood clotting factor intended to replace or supplement a specific clotting factor in a human patient, without simultaneously raising the level of other factors or, indeed, any contaminants which might produce unwanted physiological effects.

There is thus a need for an improved method of at least partially separating the vitamin K-dependent clotting factors in order to reduce or even eliminate some of the above problems. It has now been found that the vitamin K-dependent blood clotting factors can be at least partially separated from each other by metal chelate chromatography.

According to the present invention, there is provided a method for at least partially separating vitamin K-dependent blood clotting factors from a mixture containing at least one such factor, characterised in that said mixture is adsorbed on to a chelate of a polyvalent metal immobilised on an inert support, followed by elution to yield one or more fractions enriched in respect of one of said factors.

In general said mixture will contain at least two vitamin K-dependent blood clotting factors. Thus, although the invention includes purification or enrichment of fully functionalised clotting factors derived from microbiological cultures using recombinant DNA technology, the source of the clotting factors will commonly be blood plasma, particularly human plasma.

The mixture of factors to be treated in accordance with the invention will most commonly be a plasma fraction concentrate from which some plasma factors have already been removed.

In obtaining the mixed factors from plasma, typical initial fractions containing the desired components include cryoprecipitate supernatant (the supernatant remaining after cryoprecipitation of Factor VIII concentrate), Fraction I supernatant and fractions obtained after adsorption of the plasma with various affinity reagents, e.g. heparin Sepharose. In general, the factors will have been further concentrated by adsorption on to an anion exchange resin such as diethylaminoethyl (DEAE) cellulose, DEAE Sephadex or DEAE Sepharose to produce a prothrombin complex concentrate.

It should be noted that under optimal conditions for Factor IX preparation, DEAE cellulose does not bind Factor VII, although higher capacity ion-exchangers (e.g. DEAE Sephadex and DEAE Sepharose) bind Factor VII more efficiently under accepted industrial processing conditions. Thus the prothrombin complex concentrate from DEAE cellulose will not contain Factor VII. This difference in behaviour enables a concentrate containing predominantly Factor VII to be obtained, although this can also benefit from purification and enrichment according to the invention. While the anion exchange resin concentrates may beneficially be treated in accordance with the invention, other combinations of the factors may also advantageously be treated to enhance the concentration of a desired factor. In particular, an enriched fraction produced in accordance with the invention, which may contain only two of the factors, may be subjected to a second treatment to produce still further enrichment.

Existing purification systems have several inherent disadvantages. Heparin-Sepharose gives poor resolution and clotting factor activities when used on an industrial scale. Sulphated dextran produces Factor IX with a very short NAPTT (see below) which could lead to thrombotic episodes in clinical usage and dextran sulphate is cytotoxic and any leakage from such an affinity matrix may seriously contaminate the product. The present treatment has advantages over such purification systems in all these respects.

While some benefit can be obtained by a batchwise treatment of the initial mixture with the immobilised metal chelate followed by simple washing to remove the most readily eluted factor(s), the most preferred mode of operation is column chromatography. The mixture of factors can thus be applied to the top of the column and an eluant run through to provide fractions containing the separated and partially separated factors. Alternatively, the initial mixture can be applied batchwise to the immobilised metal chelate prior to loading the column.

The chelated polyvalent metals for use in the process of the invention are generally in the divalent state, Cu²⁺ being particularly preferred.

The immobilised chelating agent may comprise a conventional backbone or skeletal support (such as a support based on cellulose, polystyrene, acrylamide, silica, fluorocarbons, cross-linked dextran or cross-linked agarose) carrying groups capable of chelating polyvalent metals. Such groups are well known and can be found in text books such as Martell and Calvin, Chemistry of the Metal Chelate Compounds, Prentice Hall, Inc. New York (1952).

Iminodiacetic acid groups, -N(CH₂COOH)₂, are particuarly preferred chelating groups.

The chelating groups will generally be spaced from the backbone or skeleton of the support by linear "spacer" groupings such as optionally substituted hydrocarbon or carbohydrate chains, having advantageously from 8 to 16 atoms, e.g. 12 atoms, between the chelating group and the support. Such spacer groupings may be introduced into appropriate substrates by reaction with a divalent reagent of desired chain length. A preferred metal binding matrix is Chelating Sepharose which is a beaded 6% agarose product sold by Pharmacia AB which carries iminodiacetic acid groups, linked to the sepharose by a chain: 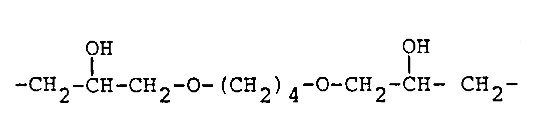
The binding of proteins to the chelating support is dependent on at least four considerations:
(i) ionic strength, where increased ionic strength apparently minimises non-specific binding;
(ii) the presence of buffer ions both in the sample to be loaded and in the equilibrated metal-chelate gel;
(iii) loading capacity of the matrix: this is a finite quantity which can be fully saturated by the more tightly binding proteins at the expense of the weaker binding proteins. The capacity of the matrix for a particular protein is thus dependent on the composition and the concentration of the protein mixture with which it is challenged during loading and the adsorption contact time;
(iv) with both binding and elution, pH is a recognised effector.

It has been found that at relatively high ionic strengths, the binding to the chelate of prothrombin (Factor II) and thrombin is reduced, while that of Factors IX and X and Protein C is increased. Since, in general, it is desirable to reduce the prothrombin level in concentrates of the other blood clotting factors, in order to avoid the possibility of thrombin formation, this finding is particularly useful. It is thus preferred to load the metal chelate in an aqueous solution of relatively high ionic strength, for example containing 0.4 to 1.0 M, preferably 0.4 to 0.6M, most preferably about 0.5 M, sodium choride and/or one or more other electrolytes providing a solution of equivalent ionic strength.

Different vitamin K-dependent clotting factors have different pH optima for binding to a chelating gel and consequently selection of the pH of the loading buffer solution provides further means for promoting preferential binding to the chelating gel of one or more vitamin K-dependent clotting factors over another such factor. Thus, for example, in the case of Cu²⁺-primed Chelating Sepharose, optimum binding of Factors II, IX and X occurs at pH 6.0-7.0, above pH 7.0 and pH 6.5-7.5 respectively. Consequently, for example, to enhance differential binding to such a gel of Factor IX over prothrombin, the loading pH will desirably be pH 7.0 or above.

Further, where it is desired to deplete prothrombin from one or more other vitamin K-dependent clotting factors, decreased binding of prothrombin in favour of other blood clotting factors, e.g. Factor IX, may be promoted by appropriate selection of the adsorption contact time and the loading challenge. Thus, for optimization of separation of Factor IX from Factor II on Cu²⁺-primed Chelating Sepharose starting with a prothrombin complex concentrate, the adsorption contact time will advantageously be over 20 minutes, more preferably about 40 minutes or longer, and the loading challenge will desirably be such that a Factor IX loading of 100-450 F.IX:Ag units per ml of gel is achieved.

Column chromatography is preferred. The initial mixture of factors is preferably applied to the top of a column, but it is also possible to load the initial mixture on to the immobilised chelate batchwise. After washings, mostly at high ionic strength, the remaining factors can be separated either by batch elution or by transferring the gel to a column for chromatographic elution.

Loading is desirably directly followed by washing with an aqueous solution of the same or similar ionic strength and pH to remove prothrombin and any thrombin which may be present. Advantageously, an acidic pH washing step may additionally be carried out to remove from the chelating gel inter-*α*-trypsin inhibitor. This protein is recognised as a major unwanted contaminant of Factor IX concentrates prepared by prior art techniques, making up in some cases as much as 30% of the total protein. It has been found, however, that inter-*α*-trypsin inhibitor can be eluted from Cu²⁺-primed Chelating Sepharose at about pH 4.5 - 5.5 without appreciable loss of Factor IX from the gel. In order to reduce electrolyte levels in the final product, it is convenient to include at least a final washing step at low ionic strength, e.g. equivalent to 100-200 mM NaCl.

The washing procedure allows for some removal of virus by additional washing steps while the protein is immobilised. Furthermore, the immobilised factors may be treated with a virucide such as a detergent at this stage and the virucide can then be removed by further washing prior to the elution step. It should be noted that the metal chelate material readily binds the protein factors of interest in the presence of detergents and/or solvents used as virucides prior to the initial adsorption according to the invention and can retain these factors when the virucide is removed by washing or indeed, when a virucidal detergent wash is applied prior to elution of the factors. This contrasts with many prior affinity columns used to adsorb such proteins, where detergent tends to affect elution of the proteins.

The clotting factors remaining on the column after removal of Factor II or, indeed, such factors applied to the column in the substantial absence of Factor II, may be eluted by a change of pH and/or use of a displacing agent selected from imidazole and amino acids, desirably at significantly lower ionic strength than the loading buffer solution in order to reduce or avoid the need to remove salts from the resulting enriched fractions. Suitable preferred ionic strengths for such elution are in the range 100-200 mM.

Where elution of desired blood clotting factors depends on a pH gradient, this may be an increasing pH gradient or a decreasing pH gradient and in either case inter-*α*-trypsin inhibitor may desirably be depleted from the chelating gel prior to collection of a Factor IX-enriched fraction. Thus, in the case of use of an increasing pH gradient for elution, it will be appreciated that the starting pH may be as low as about pH 4.5-5.5 to achieve removal of inter-*α*-trypsin inhibitor, but this will generally be followed by a substantial step upward in pH for elution of desired blood clotting factors. In particular, for example, it has been found that Factor IX- and Factor X-enriched fractions may be desirably eluted from Cu²⁺-primed Chelating Sepharose in the presence of a non-amino acid or imidazole containing buffer system at relatively low ionic strength, e.g. 100mM NaCl, by varying the pH linearly from about pH 7.0 to about pH 8.0. In the case of use of a decreasing pH gradient to elute for example from the same type of gel blood clotting factors of a prothrombin complex concentrate, following washing of the gel at above pH 7.0 at high ionic strength, e.g. 500 mM NaCl, to remove Factor II, elution of Factor X- and Factor IX-enriched fractions substantially free from inter-*α*-trypsin may desirably be achieved at relatively low ionic strength, eg. 100 mM NaCl, by stepwise decrease of the pH of the elution buffer in the acidic pH range down to about pH 4.0. In this case, the following order of protein elution is observed: Factor X/Factor V (pH about 5-5.6) inter-*α*-trypsin inhibitor/protein C (pH about 4.6), Factor IX (pH about 4.1).

If it is desired to produce purified or enriched Factor II, this may also preferably be eluted from a metal chelate column at low ionic strength in the absence of amino acids.

Where a displacing agent is employed, the concentration of imidazole or amino acid (in particular, glycine, methionine or glutamic acid) in the eluant is preferably in the range 5 to 70 mM. Suitable amino acids for use in such elution include, for example, alanine, phenylalanine, valine, lysine, glycine, methionine and glutamic acid. The last three named amino acids are particularly preferred for this purpose.

A displacing agent containing eluant is preferably buffered to a pH in the range 4 to 9, more preferably 6 to 8, e.g. about 7. Suitable buffer systems include amino alcohol buffers such as Tris and citrate-phosphate buffer. It is found that Tris enables elution of all the factors at lower concentrations of displacing agent, i.e. imidazole or amino acid.

In general, whichever eluant system is used, the order of elution, which appears to reflect binding affinity, appears to be Factor II/IIa, Factor X, Factor IX/IXa/Protein C.

In general, elution is preferably carried out in such a way that the eluted protein specific activity (purity and/or potency) is optimised. In general the concentration of the principal factor of interest, e.g. Factor IX, is preferably at least 30 iu/ml.

After elution, the solution may be freeze dried. For inactivation of virus infections, the freeze dried concentrate may be heated at, for example, 80^{o}C. We have found that the purified concentrates according to the invention substantially survive such heating, avoiding significant activation of the respective factors to a greater extent than previous concentrates.

Using the methods of the invention, it has proved possible to prepare for the first time:
(a) Factor IX substantially free of Factor II and inter-*α*-trypsin inhibitor;
(b) Protein C substantially free from Factors II, IX and X;
(c) Factor X substantially free of Factor II and having specific Factor X activity of at least 13 iu/mg protein; and
(d) Factor VII substantially free from Factor II and having a specific Factor VII activity of greater than one unit per mg protein.

### Methods of testing

The reported incidents of thrombotic episodes following infusion of prior prothrombin complex concentrate has resulted in in vitro and in vivo tests of products for their potential thrombogenic effect. The in vivo tests have been in animals (rabbits, pigs and dogs), using various criteria to asses thrombosis after infusion. In vitro tests are based upon the ability of the concentrate to reduce the clotting time of substrate plasma or fibrinogen. The significance of these tests has not been shown, but great weight is placed upon them in terms of product quality control.

The three main tests are:
NAPTT: Measures level of activated clotting factors in the sample, with an arbitrary lower limit clotting time of 150 seconds. The NAPTT cannot be related conclusively to the concentration of any particular activated clotting factor but indicates the presence of F.IXa and F.Xa.

FCT: Measures the time taken for a fibrinogen solution to clot in the presence of test material. This is a direct measure of thrombin (the penultimate protein in the blood clotting "cascade"). The lower limit clotting time is three hours, which corresponds to 5 x 10⁻³ iu/ml or 2 ng/ml of thrombin.
TGt 50: Measures the time taken to generate a known amount of thrombin. It is a measure of the activation of clotting factors which operate earlier in the process.

The following Examples are given by way of illustration only.

### Tests used in the Examples for quantification of proteins

Both functional biological activity and antigenic activity were used to quantitate the proteins of interest.

Biological activity of Factors II, IX and X was measured by established clotting assays. Unitage of clotting activity (:C) was defined using a Working Standard 87/532 which had been calibrated against the 1st International Standard for Factors II, IX and X Concentrate, code 84/681.

Biological activity of Factor VII was measured by established clotting assay or by chromogenic assay using a synthetic substrate. In both cases, unitage was assigned using an in-house Factor VII concentrate working standard which had been calibrated against a human plasma pool of greater than thirty donors, defined as having a Factor VII activity of 1.0 F.VII:C u/ml.

All antigen activities(:Ag) for Factors II, V, IX and X, Protein C and Inter-*α*-Trypsin Inhibitor were measured by Immune Electrophoresis (Laurell's Rocket method) using Normal Pooled Plasma as the standard. In each case, the standard had been assigned a potency of 1.0 unit per ml and sample activities were expressed as plasma equivalent units per ml.

In the present specification , clotting activity or "Factor -:C" is given in terms of international units per ml or iu/ml. Antigen activity or "Factor -:Ag" is given as units per ml (u/ml) or plasma equivalent units per ml (p.e.u/ml).

As the clotting and antigen activities are measures of different aspects of the protein chemistry, the international unit of clotting activity is not the same as the plasma equivalent unit. Typically, the ratio of iu : p.e.u. for Factor II, Factor IX and Factor X in partially purified protein concentrates is 0.86, 0.6 and 0.63 respectively.

### Example 1

### Preparation of Prothrombin from Prothrombin Complex Concentrate (PCC)

Chelating Sepharose was primed with copper ions by passing copper sulphate solution through it. The gel was then washed with citrate-phosphate buffer containing 500 mM NaCl at pH 7.0. PCC containing 500 mM NaCl was applied to the column at a loading of 100-200 Factor IX iu per ml gel. The protein eluate was monitored and the breakthrough protein collected. This contained prothrombin at potencies of 5-25 iu/ml with a specific activity of 5 iu/mg protein. This material was at least 70% prothrombin.

### Example 2

### Effect of ionic strength on binding of Factors II, IX, X and Protein C to copper-chelate gel.

PCC was dialysed against citrate-phosphate buffer pH 7.0 containing 100, 250, 500 or 1000 mM NaCl. This was then loaded on to a Cu²⁺-primed Chelating Sepharose column, which had been washed with the same ionic strength buffer. After loading at 430 units per ml of gel, the column was further washed with the same buffer and the eluted unbound protein assayed for Factors II, IX, X and Protein C. Table I below shows the results.

**Table I**

| Adsorption [NaCl] (mM) | Unbound units per ml of gel | | | |
|---|---|---|---|---|
| | F.II | F.IX | F.X | PC |
| 100 | 162 | 64 | 82 | 14 |
| 250 | 155 | 64 | 87 | 13 |
| 500 | 138 | 49 | 74 | 10 |
| 1000 | 95 | 35 | 55 | 7 |

Maximum differential binding of Factor IX over Factor II was observed at 500 mM NaCl.

### Example 3

### Effect of adsorption pH on binding of Factors II, IX and X to copper-chelate gel.

Chelating Sepharose was first primed with copper ions and then washed with a citrate-phosphate buffer system containing 500 mM NaCl with pH adjusted to the indicated level. PCC, containing 500 mM NaCl, was titrated to the same pH and then loaded on to the gel at about 300 Factor IX units per ml of gel. The column was further washed with the same buffer and the breakthrough/unbound protein collected and measured for Factors II, IX and X. Table II below shows the results.

**Table II**

| Adsorption pH | Unbound units per ml of gel | | |
|---|---|---|---|
| | F.II | F.IX | F.X |
| 5.5 | 231 | 69 | 121 |
| 6.0 | 227 | 64 | 93 |
| 6.5 | 189 | 64 | 74 |
| 7.0 | 234 | 71 | 69 |
| 7.5 | 285 | 43 | 75 |
| 8.2 | 328 | 59 | 155 |

Optimum binding of Factor II was achieved between pH 6.0-7.0.
Optimum binding of Factor IX was achieved above pH 7.0.
Optimum binding of Factor X was achieved between pH 6.5 and pH 7.5.
Adsorption pH can therefore be used to optimise the binding of a preferred clotting factor to the gel.

### Example 4

### Preparation of a concentrate of Factor X and Factor IX with Reduced Prothrombin

A Chelating Sepharose column was prepared and loaded as in Example 1. After application of the PCC, the column was washed with citrate-phosphate buffer containing 500 mM NaCl at pH 7.0. The ionic strength was then reduced by washing with citrate-phosphate buffer containing 100 mM NaCl. The Factor X fraction was then eluted with citrate-phosphate buffer containing 100 mM NaCl and 5 mM glycine. This contained Factor X at 15-40 Factor X iu/ml and less than 0.01 unit prothrombin per unit of Factor X. The Factor X specific activity was 13 iu/mg protein and was therefore 7% pure. This fraction also contained Factor IX in approximately equimolar amounts ( 1 iu Factor IX per unit Factor X).

### Example 5

### Preparation of Factor X with reduced Prothrombin and Factor IX

A Chelating Sepharose column was primed and loaded as described in Example 1. After application of the sample, the column was washed with citrate-phosphate buffer containing 500 mM NaCl at pH 7.0. The Factor X was then eluted with citrate-phosphate buffer at pH 7.0 containing 500 mM NaCl and 5 mM glycine. This material contained 15-30 Factor X iu/ml with a specific activity of 14 iu/mg protein. This material differed from that obtained in Example 4 by having higher prothrombin activity and lower Factor IX activity. Here, 0.25 iu/unit Factor X was obtained for both prothrombin and Factor IX. Therefore, the prothrombin still constituted about 40-50% by weight of the total protein.

### Example 6

### Preparation of a concentrate of Factor IX and Factor X with reduced Prothrombin

A Chelating Sepharose column was primed and loaded as described in Example 1. It was then washed sequentially with citrate-phosphate buffer containing first 500 mM and then 100 mM NaCl at pH 7.0. Both Factor IX and Factor X were then eluted with Tris-glycine buffer containing 100 mM NaCl, at potencies of 30 iu/ml. Prothrombin was reduced to 0.03 iu per unit of Factor IX or Factor X.

### Example 7

### Preparation of a concentrate of Factor IX and Protein C with reduced Prothrombin and Factor X

A Chelating Sepharose column was prepared and washed as described in Example 4. After elution of protein in 5 mM glycine, Tris-glycine buffer was used to elute Factor IX. It was found that Tris was suitably in the range 10-30 mM, glycine in the range 40-70 mM and NaCl 100 mM. This eluted Factor IX at potencies of 30-50 iu/ml. Prothrombin was undetectable and Factor X showed less than 0.01 unit per unit of Factor IX (less than 0.05% by weight). Protein C was also eluted at concentrations of 25-40 plasma equivalent units per ml.

### Example 8

### Separation of Protein C from Prothrombin and Factor X

Purification of Protein C is complicated by co-elution of prothrombin and Factor X in conventional chromatographic systems such as heparin-Sepharose or Dextran-Sulphate-Sepharose. However, in these systems, Factor IX contamination is minimal due to tighter binding of that protein. The metal chelate system described here can therefore be used as a step in the purification of Protein C.

The preparation and washing of a Chelating Sepharose column was as described in Example 7. As protein binding is concentration dependent, loading was as high as possible, up to 160 Factor X iu per ml gel or 240 prothrombin iu per ml of gel. The column was then washed and eluted as described in Example 7, with the Protein C eluting with the final Tris-glycine buffer eluant, separated from prothrombin and Factor X contaminants.

### Example 9

### Effect of adsorption contact time on binding and recovery of Factor II, Factor IX, Factor X and Protein C from Cu²⁺-primed Chelating Sepharose.

Chelating Sepharose was packed into columns, primed with copper ions and then washed with citrate-phosphate buffer pH 7.0 containing 500 mM NaCl. PCC, containing 500 mM NaCl, was loaded on to each column at a controlled flow rate so that the contact time with the gel could be varied in different columns. The columns were loaded at 430 Factor IX units per ml gel, washed with the 500 mM NaCl buffer, then with 100 mM NaCl buffer and then with 10mM Tris, 70 mM glycine, 100 mM NaCl buffer pH 7.0. The breakthrough and the Tris-glycine eluates were measured for Factor II, Factor IX, Factor X and Protein C. The results are shown in Table III below.

**Table III**

| Contact Time (mins) | Unbound units per ml gel | | | | Eluted units per ml gel | | | |
|---|---|---|---|---|---|---|---|---|
| | F.II | F.IX | F.X | PC | F.II | F.IX | F.X | PC |
| 10 | 172 | 156 | 153 | 27 | 91 | 231 | 135 | 66 |
| 20 | 198 | 151 | 126 | 26 | 64 | 216 | 91 | 68 |
| 40 | 189 | 63 | 66 | 13 | 39 | 242 | 160 | 78 |
| 70 | 210 | 57 | 57 | 11 | 37 | 227 | 142 | 77 |
| 110 | 247 | 72 | 105 | 18 | 23 | 266 | 126 | 93 |

Binding of protein to the gel is therefore dependent on contact time. This can be optimised for Factors IX, X and Protein C at contact times of forty minutes or greater. Increased contact time results in less Factor II binding to the gel.

### Example 10

### Effect of loading challenge on the binding and recovery of Factor II, Factor IX, Factor X and Protein C with Cu²⁺-primed Chelating Sepharose.

Chelating Sepharose was packed into several columns, primed with copper ions and washed with citrate phosphate buffer pH 7.0 containing 500 mM NaCl. PCC, containing 500 mM NaCl, was loaded on the columns in different amounts, the contact time for each column being maintained at 40 minutes by varying the flow rate through the column. The columns were then washed with buffers as described in Example 6. The results are shown in Table IV below.

**Table IV**

| Loading F.IX:Ag units per ml gel | Unbound units per ml gel | | | | Eluted units per ml gel | | | |
|---|---|---|---|---|---|---|---|---|
| | F.II | F.IX | F.X | PC | F.II | F.IX | F.X | PC |
| 43 | 6 | 0 | 0 | 0 | 13 | 30 | 23 | 9 |
| 88 | 13 | 1 | 2 | 0 | 23 | 76 | 46 | 19 |
| 173 | 54 | 15 | 18 | 2 | 42 | 112 | 85 | 37 |
| 258 | 115 | 49 | 43 | 11 | 45 | 165 | 100 | 52 |
| 344 | 185 | 55 | 52 | 11 | 31 | 186 | 115 | 62 |
| 430 | 189 | 63 | 66 | 13 | 39 | 242 | 160 | 78 |
| 513 | 305 | 175 | 128 | 42 | 26 | 256 | 136 | 79 |

Increased loading displaces Factor II preferentially. Loading challenge can be used to enhance the binding and recovery of one particular protein relative to the others. Improved separation of Factor IX from Factor II was achieved at loadings of 258-430 F.IX:Ag units per ml of gel (equivalent to approximately 150-250 F.IX:C iu per ml of gel).

### Example 11

### Effect of Factor IX concentration on binding and recovery of Factor II, Factor IX, Factor X and Protein C with Cu²⁺-primed Chelating Sepharose.

Chelating Sepharose was primed with copper ions and washed as described in previous examples. PCC containing 500 mM NaCl was diluted in citrate-phosphate buffer pH 7.0 containing 500 mM NaCl. Columns were loaded at different dilutions and washed with buffers as described in Example 6. Total loading challenge (approx. 250 units per ml of gel) and contact times were kept constant. The results are shown in Table V below.

**Table V**

| Loading potency F.IX:Ag u/ml gel | Unbound units per ml gel | | | | Eluted units per ml gel | | | |
|---|---|---|---|---|---|---|---|---|
| | F.II | F.IX | F.X | PC | F.II | F.IX | F.X | PC |
| 173 | 180 | 75 | 140 | 17 | 15 | 200 | 142 | 70 |
| 86 | 195 | 80 | 145 | 20 | 10 | 170 | 125 | 65 |
| 43 | 245 | 140 | 185 | 35 | 7 | 185 | 115 | 55 |

Higher concentrations of the loaded material are preferred for binding to the chelate to occur.

### Example 12

### Effect of chelating gel on Factor IX binding and recovery

Equal volumes of five chelating gel types were packed into five columns, primed with Cu²⁺ions and washed with citrate-phosphate buffer pH 7.0 containing 500 mM NaCl. PCC, adjusted to contain 500 mM NaCl, was loaded in equal amounts on to each column, followed by washing sequentially with the same buffer, buffer containing 100 mM NaCl and then elution with 10 mM Tris, 70 mM glycine buffer pH 7.0. Factor IX was measured in the breakthrough, wash and eluate fractions.

Gel types used were:
1. Chelating Sepharose comprising iminodiacetic acid attached to Sepharose by a 12 atom (10 carbon) spacer molecule [Pharmacia].
2. As for 1 but with additional cross-linking for Fast Flow Sepharose.
3. Agarose carrying iminodiacetic acid attached by a 12 atom (10 carbon) spacer molecule [Sigma]
4. Iminodiacetic acid directly attached to a styrene-divinyl benzene cross-linked polymer [Chelex 100, BioRad].
5. As 4 but with a coarse mesh, non-cross-linked matrix [Chelex 20, BioRad].
Table VI below shows the results.

**Table VI**

| Gel Type | Factor IX % of loaded | | |
|---|---|---|---|
| | Breakthrough | Wash | Eluate |
| 1 | 8.9 | 4.6 | 60.2 |
| 2 | 14.7 | 13.3 | 43.4 |
| 3 | 12.1 | 60.2 | 20.9 |
| 4 | 76.0 | 0.2 | 0.2 |
| 5 | 93.0 | 2.1 | 0.4 |

Factor IX binding to Cu²⁺-primed iminodiacetic groups is increased by provision of a spacer molecule between the matrix and the chelating group and by the use of non-cross-linked Chelating Sepharose.

### Example 13

### Preparation of Factor IX using Cu²⁺-primed Chelating Sepharose in a batch adsorption.

Chelating Sepharose was washed with water, then suspended in a copper sulphate solution (5mg/ml) and mixed for 20-30 minutes. The Cu²⁺-primed gel was recovered from the mixture by centrifugation and washed extensively with citrate-phosphate buffer pH 7.0 containing 500 mM NaCl. The gel was recovered as above, resuspended in a solution of PCC containing 500 mM NaCl and mixed for 20-30 minutes. The ratio of PCC to gel was approximately 150 Factor IX:C units per ml of gel. The gel was recovered by centrifugation or gravity settling and the supernatant removed.

Sequential washes with citrate-phosphate buffer pH 7.0 containing 500 mM NaCl, then 100 mM NaCl, removed weakly-bound protein and reduced the ionic strength. Factor X could then be removed by washing with 10 mM Tris, 5 mM glycine, 100 mM Nacl pH 7.0. Factor IX was subsequently eluted by washing the gel with 10 mM Tris, 70 mM glycine, 100 mM NaCl, pH 7.0.

### Example 14

### Recovery of Factor IX from Cu²⁺-primed Chelating Sepharose using different amino acid elution buffers

Columns of Chelating Sepharose were primed with Cu²⁺ ions and washed as described in previous examples. PCC (containing 500 mM NaCl) was loaded on to each column, followed by washing with citrate-phosphate buffer pH 7.0. Factor IX was recovered by eluting with 10 mM Tris, pH 7.0, containing at 70 mM an amino acid selected from (a) non-polar amino acids (e.g. alanine, valine, phenylalanine, methionine), (b) polar uncharged amino acids (e.g. glycine, serine, tyrosine, glutamine) , (c) polar negatively charged amino acids (e.g. glutamic acid) or (d) polar positively charged amino acids (e.g. lysine, arginine). Methionine, glycine and glutamic Acid were found to be particularly advantageous, these giving Factor IX yields of greater than 80% and Factor IX specific activities of greater than 5 iu/mg.

### Example 15

### Elution of Factor X and Factor IX from Chelating Sepharose using Methionine

A column of Cu²⁺-primed Chelating Sepharose was washed with citrate phosphate buffer pH 7.0 containing 500 mM NaCl. PCC was applied to the column, which was then washed with the same buffer before reducing the ionic strength by washing with a buffer containing 100 mM NaCl. Factor X was then eluted with Tris buffer pH 7.0 containing 2 mM - 5 mM methionine. The Factor X potency was greater than 15 u/ml and the specific activity was approximately 30 units/mg. Factor IX was reduced in the Factor X fraction to about 0.5 unit Factor IX per unit Factor X and prothrombin was present at less than 0.1 unit per unit Factor X.

Factor IX was found to be eluted by washing with Tris buffer containing 10-15 mM methionine. The Factor IX potency was greater than 15 u/ml and it had a specific activity of approximately 20 units/mg. Prothrombin was undetectable in the Factor IX fraction and Factor X was present at less than 0.1 Factor X unit per unit of Factor IX.

### Example 16

### Elution of Factor X and Factor IX from Chelating Sepharose using Glutamic Acid

A Cu²⁺-primed Chelating Sepharose column was prepared, washed and loaded with PCC and washed as described for methionine elution (Example 15).

Factor X was eluted with 10 mM Tris buffer containing 5 mM - 10 mM glutamic acid. The Factor X had a specific activity of 16-22 units/mg.

Factor IX, with a specific activity of approximately 15 u/mg was then eluted by raising the glutamic acid concentration in the eluting buffer to 40 mM.

### Example 17

### Preparation of Factor IX and Factor X using an increasing pH gradient to elute from Chelating Sepharose

A column of Chelating Sepharose was primed with Cu²⁺ ions and washed with buffer at pH 7.0 containing 500 mM NaCl. PCC containing 500 mM NaCl was loaded on to the column, which was then washed with buffer. Ionic strength was reduced to 100 mM NaCl using low salt buffer at pH 7.0. Some Factor X was eluted by this buffer with a specific activity of 10.5 u/mg.

The column was progressively eluted by a pH gradient from pH 7.0 to pH 8.5. Factor IX and Factor X were eluted between pH 7.1 and pH 7.9 with specific activities of 27 u/mg and 6 u/mg respectively.

### Example 18

### Preparation of Factors IX, X and Protein C using pH and glycine to elute

A column of Chelating Sepharose was primed with Cu²⁺ ions and washed with citrate-phosphate buffer, pH 7.5. PCC containing 500 mM NaCl was adjusted to pH 7.5 and loaded on to the column, which was washed with buffer. Ionic strength was then reduced by washing with citrate-phosphate buffer pH 7.5 containing 100 mM NaCl. This eluted Factor X with a specific activity of 18 u/mg in a mixture with Factor IX, which had a specific activity of 9 u/mg. A combined Protein C (15 u/ml; 6.5 units per mg protein) and Factor IX (12 u/ml; 5 units per mg protein) fraction was then collected by washing with 10 mM Tris, 5 mM glycine, 100 mM NaCl buffer, pH 8.0.

### Example 19

### Elution of protein components from Chelating Sepharose using a decreasing pH gradient

A column of Chelating Sepharose was primed with Cu²⁺ ions and washed with citrate-phosphate buffer pH 7.5 containing 500 mM NaCl. PCC was then applied after adjustment of pH to 7.5 and NaCl to 500 mM. This was followed by use of the above buffer as a wash to remove Factor II. 30% of the Factor V is the starting material was also removed in this wash. Ionic strength was then reduced by washing with buffer containing 100 mM NaCl. This also removed Factor X at a specific activity of abour 15 units per mg protein. Using citrate-phosphate buffers, a pH gradient was generated during washing of the column from pH 7.5 to pH 4.0. It was found that fractions could be collected which were rich in selected proteins. Thus, for example, at about pH 5.5, Factor X was further removed while between pH 5 and pH 5.6, a further 40% of the bound Factor V was removed from the column at about five plasma equivalent units per ml.

In this pH range, Inter-*α*-Trypsin Inhibitor also started to elute, as did Protein C. As the buffer pH fell to pH 4.6, Inter-*α*-Trypsin Inhibitor and Protein C elution continued with specific activities of 3.0 and 4.6 plasma equivalent units per mg protein respectively. Only small amounts of Factor IX were eluted during these segments of the descending pH gradient.

When the pH was then reduced further, for example to pH 4.1, Factor IX was eluted at potencies greater than 40 units per ml and specific activities of greater than 30 units per mg protein. This fraction contained no detectable Factor II or Factor X. Protein C was present at about 0.01 units per unit of Factor IX, Factor V was present at 0.06 units per unit of Factor IX and Inter-*α*-Trypsin Inhibitor was present at 0.03 units per unit of Factor IX. In terms of their contamination of one unit of Factor IX, this represents a reduction in Protein C, Factor V and Inter-*α*-Trypsin Inhibitor of 95%, 63% and 90% respectively over the starting material (PCC).

### Example 20

### Use of pH and amino acid to separate protein components from Chelating Sepharose

A column of Chelating Sepharose was primed with Cu²⁺ ions and washed with citate-phosphate buffer pH 7.5 containing 500 mM NaCl. PCC, adjusted to contain 500 mM NaCl and with a pH of 7.5, was loaded on to the column, which was then washed with the buffer described above. A further wash with citrate-phosphate buffer pH 7.5 containing 100 mM NaCl reduced the ionic strength and removed Factor X, which could be collected at a potency of at least 6 u/ml and a specific activity of at least 15 units per mg protein. The pH was then reduced by washing with a third buffer, typically citrate-phosphate pH 4.5 containing 100 mM NaCl. This removed at least 55% of the remaining bound Inter-*α*-Trypsin Inhibitor and 24% of the remaining bound Protein C, but only 5% of the bound Factor IX. The column was then re-equilibrated in a buffer having a pH and salt concentration suitable for Factor IX elution, typically pH 7.0, 100mM NaCl. The Factor IX was then eluted with an amino acid eluant buffer, e.g. glycine in Tris buffer containing 100 mM NaCl as described in previous examples. This yielded Factor IX at 200 u/ml with a specific activity of greater than 50 units per mg protein. Protein C was also present with a specific activity of 20 plasma equivalent units per mg of protein.

Compared to the starting PCC material, the Factor IX fraction contained significantly reduced amounts of contaminating proteins. Factor II, Factor X, Factor V and Inter-*α*-Trypsin Inhibitor were present per unit of Factor IX at levels of 0.0005, 0.005, 0.002 and 0.04 units respectively. This compared with values of 1.0, 1.0, 0.2 and 0.4 respectively in the PCC starting material.

### Example 21

### Preparation of Factor VII on Cu²⁺-primed Chelating Sepharose

A column of Chelating Sepharose was charged with Cu²⁺ ions, then washed with citrate-phosphate buffer pH 7.0, containing 500 mM NaCl. Factor VII concentrate, prepared by elution of DEAE-Sepharose adsorbed with cryosupernatant or Factor IX-depleted supernatant, was adjusted to contain 500 mM NaCl and loaded on to the column at 50-150 Factor VII units per ml of gel. After washing with the above buffer, the ionic strength was reduced by washing with citrate-phosphate buffer pH 7.0, containing 100 mM NaCl. The Factor VII was recovered with a specific activity of greater than one unit per mg by washing the column with 10 mM Tris, 60 mM glycine at pH 7.0 containing 100 mM NaCl.

### Example 22

### Detergent treatment of blood clotting factors while adsorbed on Chelating Sepharose

Chelating Sepharose was charged with Cu²⁺ ions washed and then loaded as described in Example 1. The column was then washed with citrate-phosphate buffer at pH 7.0 containing 500 mM NaCl. The column was then washed with at least four bed volumes of the same buffer containing 1% by weight of sodium docecyl sulphate to solubilise lipid components bound to the gel. Next, the detergent was washed from the gel with citrate-phosphate buffer containing 100 mM NaCl. This stage also served to reduce the ionic strength by desalting. Factor IX was then eluted from the gel with glycine in Tris buffer as described in Example 7. Using the same procedure, the sodium dodecyl sulphate may be replaced by the same weight of cholic acid or polyoxyethylene (20) mono-oleate (Tween 80).

### Example 23

### Freeze-drying and subsequent heat-treatment of the final fractions of Examples 1 and 4-7

Eluates of the above specified Examples were freeze-dried without loss of activity, and then heated at 80^{o}C for 72 hours to reduce potential viral infectivity. Unlike existing PCC, no thrombin generation was observed upon heating. Factors II, IX and X showed different stabilities to heating in different media. If eluates were diluted into citrate and water, heating yields of 65-90% of the unheated activity were obtained. Dilution into Tris resulted in much greater loss of activity, which was greater for Factor X than for prothrombin and greater for Factor IX than for Factor X. This general effect can be used to advantage as described below in Examples 24 and 25.

### Example 24

### Reduced Factor IX activity in a concentrate of Factor X

Example 4 and Example 6 above describe preparation of a Factor X concentrate with Factor IX also present. The Factor IX activity can be preferentially reduced by dilution of the relevant Factor X fractions into 50 mM Tris buffer pH 7.0 prior to freeze-drying. Alternatively, to avoid dilution of the eluate Factor X potency, the Tris in the eluting buffer can be increased to 50mM so that the Factor X is eluted in its formulation buffer. After freeze-drying, this material can be heated at 80°C for 72 hours. Factor IX activity will be reduced more than Factor X so that the product may be used with greater specificity as a Factor X concentrate.

### Example 25

### Heat-treatment of high purity Factor IX concentrate

In order to attain the maximum possible yields of Factor IX activity after heating, the Factor IX eluate described in Example 7 was diluted to the desired final potency in citrate or citrate-phosphate buffers or in water, freeze-dried then heat-treated at 80^{o}C for 72 hours. The eluting buffer contained Tris, but the effect of this was minimised by selection of fractions with sufficiently high Factor IX potency to permit the effective diluting out of the Tris component into the final buffer. Alternatively the formulation of the eluting buffer may be modified to allow direct freeze-drying of the undiluted eluate.

## Claims

1. A method for at least partially separating vitamin K-dependent blood clotting factors from a mixture containing at least one such factor, characterised in that said mixture is adsorbed on to a chelate of a polyvalent metal immobilised on an inert support, followed by elution to yield one or more fractions enriched in respect of one of said factors.

2. A method as claimed in claim 1, characterised in that said mixture contains a least two vitamin K-dependent blood clotting factors.

3. A method as claimed in claim 2, characterised in that said mixture is a prothrombin complex concentrate.

4. A method as claimed in claim 1, characterised in that said mixture is derived from a microbiological culture containing a vitamin K-dependent blood clotting factor produced by recombinant DNA technology.

5. A method as claimed in any one of claims 1 to 4 characterised in that said inert support has immobilised thereon chelated Cu²⁺ ions.

6. A method as claimed in claim 5 characterised in that said Cu²⁺ ions are chelated with iminodiacetic acid groups attached to an agarose support via a spacer.

7. A method as claimed in claim 6 characterised in that said spacer has from 8 to 16 atoms.

8. A method as claimed in any one of claims 1 to 7 characterised in that said mixture is adsorbed on to said chelate in the presence of an aqueous solution containing 0.4 to 1.0 M NaCl and/or one or more other electrolytes providing a solution of equivalent ionic strength.

9. A method as claimed in claim 8 characterised in that said solution contains 0.5 M NaCl and/or one or more other electrolytes providing a solution of equivalent ionic strength.

10. A method as claimed in any one of claims 1 to 9 characterised in that said mixture is adsorbed on to said chelate in the presence of a buffer solution at pH 6.0 or above.

11. A method as claimed in any one of claims 8 to 10 characterised in that a prothrombin complex concentrate is adsorbed on to an agarose support as defined in claim 6 over a period of greater than 20 minutes to achieve a Factor IX loading of 100-450 F.IX:Ag units per ml of gel.

12. A method as claimed in any one of claims 8 to 11 characterised in that adsorption of said mixture is followed by washing of the support to remove bound Factor II prior to elution of any fraction enriched in a desired further vitamin K-dependent blood clotting factor.

13. A method as claimed in any one of claims 1 to 12 characterised in that adsorption of said mixture is followed by a washing stage wherein at least the final washing step is carried out with a buffer solution containing 100-200 mM NaCl and/or one or more other electrolytes providing a solution of equivalent ionic strength.

14. A method as claimed in any of claims 1 to 13 characterised in that elution of one or more desired vitamin K-dependent blood clotting factors adsorbed on to said support is achieved by means of a pH gradient.

15. A method as claimed in any one of claims 1 to 14 characterised in that elution of one or more desired vitamin K-dependent blood clotting factors adsorbed on to said support is achieved by means of a buffer solution containing an amino acid selected from glycine, methionine and glutamic acid.

16. A method as claimed in any one of claims 1 to 15 characterised in that it includes an elution step employing an acidic pH buffer solution to specifically remove bound inter-*α*-trypsin inhibitor from the support.

17. A method as claimed in claim 16 characterised in that said elution step is followed by collection of a Factor IX-enriched fraction substantially free of said inhibitor and Factor II.

18. A method as claimed in any one of claims 1 to 17 characterised in that a Factor X-enriched fraction and/or a Factor VII-enriched fraction and/or a Protein C-enriched fraction is/are obtained, substantially free of Factor II.

19. A method as claimed in any one of the preceding claims characterised in that column chromatography is performed.

20. A method as claimed in claim 1 wherein Factor IX is at least partially separated from a mixture containing Factor IX and at least one other vitamin K-dependent blood clotting factor.

21. A method as claimed in claim 1 wherein Factor IX is at least partially separated from a mixture containing Factor IX and at least one other vitamin K-dependent blood clotting factor and wherein said chelated polyvalent metal is copper.

22. A method as claimed in claim 21 wherein Factor IX is eluted from immobilised chelated copper by means of a buffer solution containing an amino acid selected from glycine, methionine and glutamic acid.

## Patentansprüche

1. Verfahren um Vitamin-K-abhängige Blutgerinnungsfaktoren aus einer zumindest einen derartigen Faktor enthaltenden Mischung zumindest teilweise abzutrennen, dadurch gekennzeichnet, daß diese Mischung an ein Chelat eines mehrwertigen Metalls adsorbiert wird, das an einem inerten Träger immobilisiert ist, woran sich eine Elution anschließt, um eine oder mehrere hinsichtlich eines dieser Faktoren angereicherte Fraktionen zu erhalten.

2. Verfahren gemäß Anspruch 1, dadurch gekennzeichnet, daß die Mischung zumindest zwei Vitamin-K-abhängige Blutgerinnungsfaktoren enthält.

3. Verfahren gemäß Anspruch 2, dadurch gekennzeichnet, daß diese Mischung ein Prothrombinkomplex-Konzentrat ist.

4. Verfahren gemäß Anspruch 1, dadurch gekennzeichnet, daß diese Mischung einer mikrobiologischen Kultur entstammt, die einen durch rekombinante DNA-Technologie gebildeten Vitamin-K-abhängigen Blutgerinnungsfaktor enthält.

5. Verfahren gemäß einem der Ansprüche 1 bis 4, dadurch gekennzeichnet, daß der inerte Träger immobilisiert hierauf chelatisierte Cu²⁺-Ionen aufweist.

6. Verfahren gemäß Anspruch 5, dadurch gekennzeichnet, daß die Cu²⁺-Ionen mit Iminodiessigsäuregruppen, die an einen Agarose-Träger über einen Spacer geknüpft sind, chelatisiert sind.

7. Verfahren gemäß Anspruch 6, dadurch gekennzeichnet, daß der Spacer 8 bis 16 Atome aufweist.

8. Verfahren gemäß einem der Ansprüche 1 bis 7, dadurch gekennzeichnet, daß die Mischung an dem Chelat in Gegenwart einer wäßrigen Lösung adsorbiert wird, die 0,4 bis 1,0 M NaCl und/oder einen oder mehrere andere Elektrolyten zur Schaffung einer Lösung mit einer äquivalenten Ionenstärke enthält.

9. Verfahren gemäß Anspruch 8, dadurch gekennzeichnet, daß die Lösung 0,5 M NaCl und/oder einen oder mehrere andere Elektrolyten zur Schaffung einer Lösung mit einer äquivalenten Ionenstärke enthält.

10. Verfahren gemäß einem der Ansprüche 1 bis 9, dadurch gekennzeichnet, daß die Mischung an dem Chelat in Gegenwart einer Pufferlösung bei pH 6,0 oder darüber adsorbiert wird.

11. Verfahren gemäß einem der Ansprüche 8 bis 10, dadurch gekennzeichnet, daß ein Prothrombinkomplex-Konzentrat an einem Agarose-Träger wie in Anspruch 6 definiert über einen Zeitraum von mehr als 20 Minuten adsorbiert wird, um eine Faktor IX-Beladung von 100 bis 450 F.IX:Ag-Einheiten je ml Gel zu ergeben.

12. Verfahren gemäß einem der Ansprüche 8 bis 11, dadurch gekennzeichnet, daß sich an die Adsorption der Mischung ein Waschen des Trägers zur Entfernung von gebundenem Faktor II vor der Elution irgendeiner Fraktion, die an einem gewünschten weiteren Vitamin-K-abhängigen Blutgerinnungsfaktor angereichert ist, anschließt.

13. Verfahren gemäß einem der Ansprüche 1 bis 12, dadurch gekennzeichnet, daß sich an die Adsorption der Mischung eine Waschstufe anschließt, bei der zumindest die letzte Waschstufe mit einer Pufferlösung durchgeführt wird, die 100 - 200 mM NaCl und/oder einen oder mehrere weitere Elektrolyten für die Schaffung einer Lösung mit äquivalenter Ionenstärke enthält.

14. Verfahren gemäß einem der Ansprüche 1 bis 13, dadurch gekennzeichnet, daß die Elution von einem oder mehreren gewünschten Vitamin-K-abhängigen Blutgerinnungsfaktoren die an dem Träger adsorbiert sind, mit Hilfe eines pH-Gradienten erzielt wird.

15. Verfahren gemäß einem der Ansprüche 1 bis 14, dadurch gekennzeichnet, daß die Elution von einem oder mehreren gewünschten Vitamin-K-abhängigen Blutgerinnungsfaktoren, die an dem Träger adsorbiert sind, mit Hilfe einer Pufferlösung erzielt wird, die eine Aminosäure, ausgewählt unter Glycin, Methionin und Glutaminsäure enthält.

16. Verfahren gemäß einem der Ansprüche 1 bis 15, dadurch gekennzeichnet, daß es eine Elutionsstufe unter Verwendung einer Pufferlösung mit saurem pH umfaßt, um gebundenen Inter-*α*-Trypsin-Inhibitor spezifisch aus dem Träger zu entfernen.

17. Verfahren gemäß Anspruch 16, dadurch gekennzeichnet, daß sich an die Elutionsstufe ein Sammeln einer hinsichtlich Faktor IX angereicherten Fraktion, die im wesentlichen von dem Inhibitor und Faktor II frei ist, anschließt.

18. Verfahren gemäß einem der Ansprüche 1 bis 17, dadurch gekennzeichnet, daß man eine Faktor X-angereicherte Fraktion und/oder eine Faktor VII-angereicherte Fraktion und/oder eine Protein C-angereicherte Fraktion erhält, welche im wesentlichen von Faktor II frei ist/sind.

19. Verfahren gemäß einem der vorhergehenden Ansprüche, dadurch gekennzeichnet, daß man eine Säulenchromatographie durchführt.

20. Verfahren gemäß Anspruch 1, worin Faktor IX zumindest teilweise aus einer Mischung abgetrennt wird, die Faktor IX und zumindest einen anderen Vitamin-K-abhängigen Blutgerinnungsfaktor enthält.

21. Verfahren gemäß Anspruch 1, worin Faktor IX zumindest teilweise aus einer Mischung abgetrennt wird, die Faktor IX und zumindest einen anderen Vitamin-K-abhängigen Blutgerinnungsfaktor enthält, und worin das chelatisierte mehrwertige Metall Kupfer ist.

22. Verfahren gemäß Anspruch 21, worin Faktor IX aus immobilisiertem chelatisierten Kupfer mit Hilfe einer Pufferlösung eluiert wird, die eine Aminosäure, ausgewählt unter Glycin, Methionin und Glutaminsäure enthält.

## Revendications

1. Procédé pour séparer au moins partiellement les facteurs de coagulation du sang dépendants de la vitamine K d'un mélange contenant au moins un tel facteur, caractérisé en ce que le mélange est adsorbé sur un chélate d'un métal polyvalent immobilisé sur un support inerte, puis est élué pour donner une ou plusieurs fractions enrichies en l'un de ces facteurs.

2. Procédé suivant la revendication 1, caractérisé en ce que le mélange contient au moins deux facteurs de coagulation du sang dépendants de la vitamine K.

3. Procédé suivant la revendication 2, caractérisé en ce que le mélange est un concentré de complexe de prothrombine.

4. Procédé suivant la revendication 1, caractérisé en ce que ce mélange dérive d'une culture microbiologique contenant un facteur de coagulation du sang dépendant de la vitamine K produit par la technologie de la DNA recombinant.

5. Procédé suivant l'une quelconque des revendications 1 à 4, caractérisé en ce que le support inerte porte des ions Cu²⁺ chélatés immobilisés.

6. Procédé suivant la revendication 5, caractérisé en ce que les ions Cu²⁺ sont chélatés par des radicaux acide iminodiacétique attachés à un support d'agarose par un espaceur.

7. Procédé suivant la revendication 6, caractérisé en ce que l'espaceur compte 8 à 16 atomes.

8. Procédé suivant l'une quelconque des revendications 1 à 7, caractérisé en ce que le mélange est adsorbé sur le chélate en présence d'une solution aqueuse contenant du NaCl 0,4 à 1,0 M et/ou un ou plusieurs autres électrolytes formant une solution de force ionique équivalente.

9. Procédé suivant la revendication 8, caractérisé en ce que la solution contient du NaCl 0,5 M et/ou un ou plusieurs autres électrolytes formant une solution de force ionique équivalente.

10. Procédé suivant l'une quelconque des revendications 1 à 9, caractérisé en ce que le mélange est adsorbé sur le chélate en présence d'une solution tampon à un pH de 6,0 ou plus élevé.

11. Procédé suivant l'une quelconque des revendications 8 à 10, caractérisé en ce qu'un concentré de complexe de prothrombine est adsorbé sur un support d'agarose tel que défini dans la revendication 6 en une durée supérieure à 20 minutes pour arriver à un chargement de Facteur IX de 100 à 450 Unités F.IX:Ag par ml de gel.

12. Procédé suivant l'une quelconque des revendications 8 à 11, caractérisé en ce que l'adsorption du mélange est suivie d'un lavage du support pour éliminer le Facteur II lié, avant l'élution de toute fraction enrichie en un autre facteur de coagulation du sang dépendant de la vitamine K souhaité.

13. Procédé suivant l'une quelconque des revendications 1 à 12, caractérisé en ce que l'adsorption du mélange est suivie d'un stade de lavage au cours duquel au moins l'étape de lavage finale est exécutée avec une solution tampon contenant du NaCl 100 à 200 mM et/ou un ou plusieurs autres électrolytes formant une solution de force ionique équivalente.

14. Procédé suivant l'une quelconque des revendications 1 à 13, caractérisé en ce que l'élution d'un ou plusieurs facteurs de coagulation du sang dépendants de la vitamine K désirés adsorbés sur le support est effectuée à l'aide d'un gradient de pH.

15. Procédé suivant l'une quelconque des revendications 1 à 14, caractérisé en ce que l'élution d'un ou plusieurs facteurs de coagulation du sang dépendants de la vitamine K désirés adsorbés sur le support est effectuée à l'aide d'une solution tampon contenant un acide aminé choisi entre la glycine, la méthionine et l'acide glutamique.

16. Procédé suivant l'une quelconque des revendications 1 à 15, caractérisé en ce qu'il comprend une étape d'élution mettant en jeu une solution tampon de pH acide pour éliminer spécifiquement du support l'inhibiteur d'inter-*α*-trypsine lié.

17. Procédé suivant la revendication 16, caractérisé en ce que l'étape d'élution est suivie de la collecte d'une fraction enrichie en Facteur IX sensiblement exempte de l'inhibiteur et du Facteur II.

18. Procédé suivant l'une quelconque des revendications 1 à 17, caractérisé en ce qu'une fraction enrichie en Facteur X et/ou une fraction enrichie en Facteur VII et/ou une fraction enrichie en Protéine C sont obtenues sensiblement exemptes du Facteur II.

19. Procédé suivant l'une quelconque des revendications précédentes, caractérisé en ce qu'une chromatographie sur colonne est exécutée.

20. Procédé suivant la revendication 1, dans lequel le Facteur IX est au moins partiellement séparé d'un mélange contenant le Facteur IX et au moins un autre facteur de coagulation du sang dépendant de la vitamine K.

21. Procédé suivant la revendication 1, dans lequel le Facteur IX est au moins partiellement séparé d'un mélange contenant le Facteur IX et au moins un autre facteur de coagulation du sang dépendant de la vitamine K et dans lequel le métal polyvalent chélaté est le cuivre.

22. Procédé suivant la revendication 1, dans lequel le Facteur IX est élué du cuivre chélaté immobilisé à l'aide d'une solution tampon contenant un acide aminé choisi entre la glycine, la méthionine et l'acide glutamique.
